# EUROPEAN PATENT APPLICATION

(11) **EP 2 022 496 A1**
(43) Date of publication of application: **11.02.2009**
(21) Application number: 07112558.7
(22) Date of filing: 16.07.2007
(51) Int. Cl.: A61K 31/4045, A61K 9/16, A61K 9/20, A61K 9/28, A61P 25/14, A61P 25/16

(54) **Stable ropinirole compositions**

(71) Applicant: Ranbaxy Laboratories Limited, Gurgaon, Haryana 122001 (IN)
(72) Inventor: Bhargava, Ankur, Gurgaon 122001, Haryana (IN); Suseendharnath, Amarnath, Gurgaon 122001, Haryana (IN); Mathur, Rajeev Shankar, 122002, Haryana (IN); Rampal, Ashok, 143001, Punjab (IN)
(74) Representative: Cronin, Brian Harold John

(57) **Abstract**

The present invention relates to stable ropinirole compositions and processes of preparation thereof. Stable ropinirole compositions comprise unmicronised ropinirole and one or more pharmaceutically acceptable excipients, wherein the compositions are prepared by direct compression and is devoid of colloidal silicon dioxide and talc. The compositions are useful for treating signs and symptoms of idiopathic Parkinson's disease and for treating moderate-to-severe primary Restless Legs Syndrome.

## Description

### Field of the Invention

The present invention relates to stable ropinirole compositions and processes of preparation thereof. Stable ropinirole compositions comprise unmicronised ropinirole and one or more pharmaceutically acceptable excipients, wherein the composition is prepared by direct compression and is devoid of colloidal silicon dioxide and talc. The compositions are useful for treating signs and symptoms of idiopathic Parkinson's disease and for treating moderate-to-severe primary Restless Legs Syndrome.

### Background of the Invention

Ropinirole hydrochloride {4-[2-(dipropylamino)ethyl]-1,3-dihydro-2H-indol-2-one monohydrochloride}is disclosed in U.S. Patent No. 4,452,808 and is a non-ergoline dopamine agonist that can be orally administered to treat Parkinson;s disease.

Parkinson's disease is a debilitating disease characterized by disturbance of voluntary movement in which muscles become stiff and sluggish, movement becomes clumsy and difficult, and uncontrollable rhythmic twitching of groups of muscles produces characteristic shaking or tremor. The condition is believed to be caused by the degeneration of dopaminergic neurons in the nigral striatal system of the brain, leading to inadequate release of the neurotransmitter dopamine.

Ropinirole, an indolone derivative, alleviates the deficiencies of conventional anti-parkinsonian drugs such as L-DOPA and ergot alkaloids, and in particular has reduced risk of dyskinesia. Ropinirole has additional beneficial effects on the central nervous system (namely, it has anti-depressant and anxiolytic effects), particularly since patients requiring current therapies often need to take separate anti-depressant medication. Such beneficial effects from a single compound obviate the need for separate therapies. Ropinirole is believed to act by stimulation of post-synaptic dopamine D2-type receptors within the caudate putamen of the brain, leading to an increase in the activity of the neurotransmitter dopamine.

Ropinirole is typically in the form of its hydrochloride salt when used in tablets. Ropinirole hydrochloride has been provided as an immediate-release formulation or a 24-hour controlled-release formulation.

PCT Patent Application No. WO 91/16306 discloses the process for preparing ropinirole. PCT Patent Application No. WO 01/78688 discloses a multi-layer controlled-release tablet comprising a mixed matrix of hydrophilic or lipophilic components to control the release rate of one or more therapeutically active agents from the tablets. PCT Patent Application No. WO 05/018605 discloses a controlled-release oral dosage form of ropinirole for oral administration and to its use in treating diseases that prevent or disturb sleep, particularly Restless Legs Syndrome (RLS).

Stability of a drug is of paramount importance. Effects on the stability of ropinirole compositions vary with changes in certain parameters of such compositions and processes of preparation thereof.

The prior art does not acknowledge the criticality of any stability parameters of ropinirole compositions and thus there is a need to prepare stable ropinirole compositions that exhibits enhanced or comparable stability in view of existing ropinirole compositions.

We have surprisingly found that particle size of ropinirole plays a major role in the stability of the composition. Further, selection of particular pharmaceutically acceptable excipients and the process of preparing the composition were found to affect stability.

Micronization processes reduce the size of solid drug particles to micron-sized particles and is commonly done by spray drying or use of air attrition methods. Micronization increases particle surface area, allowing greater interaction with excipients. Compositions having unmicronised ropinirole have been observed to be relatively stable when compared to the compositions having micronised ropinirole.

Direct compression is advantageous over wet granulation because the technique is simple and economical. In addition, it was observed that ropinirole compositions prepared by direct compression were effectively more stable when compared with compositions prepared by wet granulation.

Further, we have surprisingly found that addition of certain pharmaceutically acceptable excipients adversely affects the stability of the composition.

Thus, the present invention relates to stable ropinirole compositions and process of preparing thereof. The compositions have acceptable stability in terms of related substances, even after storage under accelerated conditions. Further, the compositions are cost-effective and easy to manufacture on a commercial scale without requiring complex processing steps.

### Summary of the Invention

In one aspect, provided are stable ropinirole compositions comprising unmicronised ropinirole and one or more pharmaceutically acceptable excipients. The compositions may include one or more of the following embodiments. For example, the unmicronised ropinirole can have a particle size having a D₉₀ value from 50 to 200 microns, a D₅₀ value from 20 to 80 microns and a D₁₀ value from 5 to 30 microns.

In another embodiment, the one or more pharmaceutically acceptable excipients can be selected from one or more of binders, diluents, disintegrants, lubricants or mixtures thereof. Suitable binders can be selected from one or more cellulose derivatives, gums, water-soluble vinylpyrrolidone polymers, sugars or mixtures thereof. Suitable diluents can be selected from one or more sugars, sugar alcohols, cellulose derivatives, starches or mixtures thereof. Suitable disintegrants can be selected from one or more sodium starch glycolate, croscarmellose sodium, crospovidone and corn starch. Suitable lubricants can be selected from one or more of magnesium stearate, talc, sodium stearyl fumarate and colloidal silicon dioxide.

In another embodiment, the compositions can be prepared by direct compression.

In another aspect, provided are stable ropinirole compositions comprising ropinirole and one or more pharmaceutically acceptable excipients wherein the composition is devoid of colloidal silicon dioxide. These compositions can include one or more of the following embodiments. For example in one embodiment, the composition is devoid of talc.

In another embodiment, the ropinirole is unmicronised ropinirole. In yet another embodiment, the unmicronised ropinirole can have a particle size having a D₉₀ value from 50 to 200 microns, a D₅₀ value from 20 to 80 microns and a D₁₀ value from 5 to 30 microns.

In one embodiment, the composition can be prepared by direct compression.

In another embodiment, the one or more pharmaceutically acceptable excipients can be selected from one or more binders, diluents, disintegrants, lubricants or mixtures thereof. Suitable binders can be selected from one or more cellulose derivatives, gums, water-soluble vinylpyrrolidone polymers, sugars or mixtures thereof. Suitable diluents can be selected from one or more sugars, sugar alcohols, cellulose derivatives, starches or mixtures thereof. Suitable disintegrants can be selected from one or more of sodium starch glycolate, croscarmellose sodium, crospovidone and corn starch. Suitable lubricants are selected from one or more of magnesium stearate, talc, sodium stearyl fumarate and colloidal silicon dioxide.

In yet another aspect, provided is a process for preparing a stable ropinirole composition comprising the steps of:
(a) preparing a dry blend of ropinirole and one or more pharmaceutically acceptable excipients, and
(b) directly compressing the dry blend of step (a) to form a stable ropinirole composition.

In one embodiment of the process, the ropinirole is unmicronised ropinirole.

### Detailed Description of the Invention

In one aspect, provided herein are stable ropinirole compositions.

According to one embodiment, stable ropinirole compositions comprise unmicronised ropinirole and one or more pharmaceutically acceptable excipients. It was observed that compositions containing unmicronised ropinirole provide improved stability when compared to compositions containing micronised ropinirole.

The term "ropinirole," as used herein, refers to ropinirole or salts thereof.
Preferably, ropinirole is in the form of ropinirole hydrochloride.

The term "unmicronised ropinirole," as used herein, refers to particles of ropinirole, wherein the D₉₀ value ranges from 50 to 200 microns, the D₅₀ value ranges from 20 to 80 microns and the D₁₀ value ranges from 5 to 30 microns.

The term "composition," as used herein, refers to any oral dosage form, including but not limited to tablets or capsules. Composition stability is measured in terms of total related substances as determined by HPLC or TLC.

Suitable pharmaceutically acceptable excipients may be selected from one or more of binders, diluents, disintegrants, lubricants/glidants or mixtures thereof.

Suitable binders include polymeric substances having sufficient elasticity and structural stability as a film. Generally, binders may be selected from one or more of cellulose derivatives (such as hydroxypropylmethyl cellulose, hydroxypropyl cellulose and methylcellulose), gums (such as xanthan gum, gum acacia and tragacanth), water-soluble vinylpyrrolidone polymers (such as polyvinylpyrrolidone and copolymer of vinylpyrrolidone vinyl acetate), sugars (such as sorbitol and mannitol) or mixtures thereof.

Suitable diluents may be selected from one or more of sugars (such as dextrose, glucose and lactose); sugar alcohols (such as sorbitol, xylitol and mannitol); cellulose derivatives (such as powdered cellulose and microcrystalline cellulose); starches (such as corn starch, pregelatinized starch and maize starch) or mixtures thereof.

Suitable disintegrants may be selected from one or more of sodium starch glycolate, croscarmellose sodium, crospovidone, corn starch or mixtures thereof.

Suitable lubricant/glidants may be selected from one or more of magnesium stearate, talc, sodium stearyl fumarate, colloidal silicon dioxide or mixtures thereof.

Tablet or capsule compositions may be prepared by conventional processes known to one of ordinary skill in the art, including wet granulation, dry granulation or direct compression. Preferably, such processes involve direct compression. Surprisingly, it was observed that compositions prepared by direct compression were stable in terms of related substances when compared to compositions prepared by wet granulation. Direct compression is a dry process, which avoids the use of water.

According to one embodiment, there is provided a process for the preparation of stable ropinirole compositions comprising the steps of: (a) preparing a dry blend of ropinirole and one or more pharmaceutically acceptable excipients, and b) directly compressing the dry blend of step (a) to obtain a stable ropinirole composition.

Further, it was observed that ropinirole compositions comprising unmicronised ropinirole and prepared by direct compression provide a stable composition.

Thus according to another embodiment, there is provided a process for the preparation of stable ropinirole compositions, the process comprising the steps of: (a) preparing a dry blend of unmicronised ropinirole and one or more pharmaceutically acceptable excipients, and b) directly compressing the blend of step (a) to obtain stable ropinirole composition.

Further, it was observed that the presence of certain pharmaceutically acceptable excipients e.g. talc and colloidal silicon dioxide have adverse effect on the stability of the composition.

Thus according to another embodiment there is provided a stable ropinirole composition comprising unmicronised ropinirole and one or more pharmaceutically acceptable excipients, wherein the composition is prepared by direct compression and is devoid of colloidal silicon dioxide.

According to another embodiment, there is provided a stable ropinirole composition comprising unmicronised ropinirole and one or more pharmaceutically acceptable excipients, wherein the composition is prepared by direct compression and is devoid of colloidal silicon dioxide and talc.

The stable composition of ropinirole may optionally be coated with non-functional layers comprising film-forming polymers, if desired.

Suitable film-forming polymers include one or more of ethyl cellulose, hydroxypropyl methyl cellulose, hydroxypropyl cellulose, methylcellulose, carboxymethyl cellulose, hydroxymethylcellulose, hydroxyethylcellulose, cellulose acetate, hydroxypropyl methylcellulose phthalate, cellulose acetate phthalate; waxes (such as polyethylene glycol); methacrylic acid polymers (such as Eudragit RL and RS); and the like or mixtures thereof. Alternatively, commercially available coating compositions may be used for the coating including film-forming polymers marketed under various names, such as, Opadry.

While the present invention has been described in terms of its specific embodiments, certain modifications and equivalents will be apparent to those skilled in the art and are included within the scope of the present invention.

### Examples

### Examples 1 - 2: Ropinirole Compositions

| **Ingredient** | **Example 1** | **Example 2** |
|---|---|---|
| | mg/tab | mg/tab |
| Ropinirole HCl | 0.285 | 0.285 |
| Lactose monohydrate (lactose 200M) | 15.0 | 15.0 |
| Microcrystalline cellulose | 70.0 | 70.0 |
| Lactose monohydrate (DCI 11) | 56.2 | 56.2 |
| Croscarmellose sodium | 4.5 | 4.5 |
| Colloidal anhydrous silica | 1.0 | 1.0 |
| Talc | 1.5 | 1.5 |
| Magnesium stearate | 1.5 | 1.5 |
| **TOTAL** | 150 | 150 |
| Opadry film coating | 4.5 | 4.5 |
| Particle size of ropinirole HCl | Micronised* | Unmicronised** |
| **Process** | Direct Compression | Direct Compression |

| | | |
|---|---|---|
| * D₉₀ = 6µm, D₅₀ = 3µm, D₁₀= 1µm **D₉₀ = 108µm, D₅₀ = 40µm, D₁₀=16µm | | |

### Process of Preparation

1. Ropinirole, lactose (lactose 200M), microcrystalline cellulose, croscarmellose sodium, lactose (DCI 11) and colloidal anhydrous silica were all sifted and mixed to obtain a blend.
2. Magnesium stearate and talc were then mixed with the blend obtained in step 1 to obtain a final blend followed by compression to obtain tablets.
3. Tablets obtained in step 2 were then coated with Opadry film coating to obtain coated tablets.

The compositions of Example 1 and Example 2 were evaluated for total related substances (both at initial time point and after storage). The results are provided in Table 1 below.

**Table 1:**

| | **Example 1** | | **Example 2** | |
|---|---|---|---|---|
| **Particle size of ropinirole HCl** | **Micronised** | | **Unmicronised** | |
| **Process** | Direct Compression | | Direct Compression | |
| | **Initial** | **6M** | **Initial** | **6M** |
| | | (40°C/75% RH, Alu-Alu pack) | | (40°C/75% RH, Alu-Alu pack) |
| Total RS | 1.82 | 9.864 | 0.696 | 2.469 |

As evident from the above data, the composition of Example 1 having micronised ropinirole showed higher total related substances in comparison to the composition of Example 2 having unmicronised ropinirole. The composition of Example 2 showed higher stability compared to the composition of Example 1 by measurement of total related substances.

### Examples 3 - 4: Ropinirole Compositions

| **Example** | **Example 3** | **Example 4** |
|---|---|---|
| Ingredients | mg/tab | mg/tab |
| Ropinirole HCl | 0.285 | 0.285 |
| Lactose monohydrate (Pharmatose 200M) | 15.0 | 15.0 |
| Microcrystalline cellulose | 70.0 | 70.0 |
| Lactose (DCI 11) | 58.715 | 58.715 |
| Croscarmellose sodium | 4.5 | 4.5 |
| Magnesium stearate | 1.5 | 1.5 |
| **TOTAL** | 150 | 150 |
| Opadry film coating | 4.5 | 4.5 |
| Particle size of ropinirole HCl | Micronised* | Unmicronised** |
| **Process** | Direct Compression | Direct Compression |

| | | |
|---|---|---|
| *D₉₀ = 21µm, D₅₀ =10µm, D₁₀= 2µm **D₉₀ = 67µm, D₅₀ =30µm, D₁₀= 9µm | | |

### Process of Preparation

1. Ropinirole hydrochloride, lactose monohydrate (pharmatose 200M), microcrystalline cellulose, lactose (DCI 11) and croscarmellose sodium were all sieved and mixed to obtain a blend
2. Magnesium stearate was then mixed with the blend obtained in step 1 to obtain a final blend, which was then compressed to obtain tablets.
3. Tablets obtained in step 2 were then coated with Opadry film coating.

The compositions of Example 3 and Example 4 were evaluated for total related substances (both at initial time point and after storage). The results are provided in Table 2 below.

**Table 2:**

| **Example** | **Example 3** | | **Example 4** | |
|---|---|---|---|---|
| **Particle size of ropinirole HCl** | **Micronised** | | **Unmicronised** | |
| **Process** | Direct Compression | | Direct Compression | |
| | **Initial** | **15D**(60° C, HDPE bottle) | **Initial** | 15D(60°C,HDPE bottle) |
| Total RS | 0.151 | 1.635 | 0.08 | 0.205 |

The above results further show that compositions having micronised ropinirole have higher amount of total related substances in comparison to composition with unmicronised ropinirole. The composition of Example 4 has higher stability when compared to the composition of Example 1 by measurement of total related substances.

The composition of Example 4 was also subjected to longer periods of storage. The amount of related substances after storage is provided in Table 3 below:

**Table 3**

| | **Example 4** | |
|---|---|---|
| **Particle size of ropinirole HCl** | **Unmicronised** | |
| **Process** | Direct Compression | |
| | **Initial** | **6M** (40° / 75% RH, Aclar-Alu Blister) |
| Total RS | 0.080 | 1.920 |
| LOD | 3.96 | 2.94 |

The above results show that composition having unmicronised ropinirole are stable even after storage at accelerated conditions for a period of 6 months.

### Examples 5-7: Ropinirole Compositions

| **Ingredients** | **Example 5** | **Example 6** | **Example 7** |
|---|---|---|---|
| | mg/tab | mg/tab | mg/tab |
| Ropinirole HCl | 0.285 | 0.285 | 0.285 |
| Lactose monohydrate (pharmatose 200M) | 15.0 | 15.0 | 15.0 |
| Microcrystalline cellulose | 70.0 | 70.0 | 70.0 |
| Lactose (DCI 11) | 56.2 | 56.2 | 56.2 |
| Croscarmellose sodium | 4.5 | 4.5 | 4.5 |
| Colloidal silicon dioxide | 1.0 | - | - |
| Talc | 1.5 | 1.5 | - |
| Magnesium Stearate | 1.5 | 1.5 | 1.5 |
| TOTAL | 150.0 | 150.0 | 150.0 |
| **API** | Micronised* | Micronised* | Micronised* |
| **Process** | Direct Compression | Direct Compression | Direct Compression |

| | | | |
|---|---|---|---|
| * D₉₀ = 21µm, D₅₀ =10µm, D₁₀= 2µm | | | |

### Process of Preparation for Compositions of Example 5 and 6

1. Ropinirole, lactose (pharmatose 200M), microcrystalline cellulose, lactose (DCI 11), croscarmellose sodium and colloidal anhydrous silica were all sifted and mixed to obtain a blend.
2. Magnesium stearate and talc were then mixed with the blend obtained in step 1 to obtain a final blend followed by compression to obtain tablets.

### Process of Preparation for Compositions of Example 7

1. Ropinirole, lactose (lactose 200M), microcrystalline cellulose, lactose (DCI 11) and croscarmellose sodium were all sifted and mixed to obtain a blend.
2. Magnesium stearate was then mixed with the blend obtained in step 1 to obtain a final blend followed by compression to obtain tablets.

The compositions of Example 5, Example 6 and Example 7 were evaluated for total related substances (both at initial time point and after storage). The results are provided in Table 4 below.

**Table 4:**

| | **Example 5** | | **Example 6** | | **Example 7** | |
|---|---|---|---|---|---|---|
| Particle size of ropinirole HCl | micronised | | micronised | | micronised | |
| **Process** | Direct Compression | | Direct Compression | | Direct Compression | |
| | **Initial** | **15D** | **Initial** | **15D** | **Initial** | **15D** |
| | | (60°C, HDPE bottle) | | (60°C, HDPE bottle) | | (60°C,HD PE bottle) |
| Total RS | 0.341 | 5.655 | 0.157 | 1.318 | 0.137 | 0.975 |

The above results show the effect of talc and colloidal silicon dioxide on the stability of the composition. The composition of Example 7, without talc and colloidal silicon dioxide, shows lower total related substances when compared with the compositions of Example 6 (with talc) and Example 5 (with talc and colloidal silicon dioxide).

### Example 8: Ropinirole Composition and Stability Comparison

| | **Example 8** | **Example 2** |
|---|---|---|
| **Ingredients** | **mg/tab** | **mg/tab** |
| **Intragranular** | | |
| Ropinirole HCl | 0.285 | 0.285 |
| Lactose monohydrate (pharmatose 200M) | 100 | 15.0 |
| Microcrystalline cellulose (Avicel 102) | 41.22 | 70.0 |
| Lactose monohydrate (DCI 11) | - | 56.2 |
| Croscarmellose sodium | - | 4.5 |
| Purified Water | q.s. | - |

| **Extragranular** | | |
|---|---|---|
| Croscarmellose sodium | 4.5 | - |
| Colloidal silicon dioxide | 1 | 1.0 |
| Talc | 1.5 | 1.5 |
| Magnesium stearate | 1.5 | 1.5 |
| **TOTAL** | 150 | 150 |
| Opadry film coating | 4.5 | 4.5 |
| **Particle size of ropinirole HCl** | **Unmicronised*** | **Unmicronised*** |
| **Process** | **Wet Granulation** | **Direct Compression** |

| | | |
|---|---|---|
| * D₉₀ = 108µm, D₅₀ = 40µm, D₁₀= 16µm | | |

### Process of Preparation for Composition of Example 8

1. Ropinirole hydrochloride, lactose and microcrystalline cellulose were all sifted and were mixed in a rapid mixer granulator to obtain a blend.
2. The blend obtained in step 1 was granulated with purified water to obtain granules.
3. The granules obtained in step 2 were dried in fluidized bed drier and sifted to obtain dry granules.
4. Croscarmellose sodium and colloidal silicon dioxide were then sifted and mixed with the dry granules of step 3 to obtain a mixture.
5. Talc was sifted and added to mixture obtained in step 4 to obtain final blend.
6. The final blend obtained in step 5 was then compressed to obtain tablets.

The compositions of Example 8 and Example 2 were evaluated for total related substances (both at initial time point and after storage). The results are provided in Table 5 below.

**Table 5: Comparison of Compositions of Example 8 and Example 2**

| | **Example 8** | | **Example 2** | |
|---|---|---|---|---|
| **Particle size of ropinirole HCl** | **Unmicronised** | | **Unmicronised** | |
| **Process** | Wet granulation | | Direct Compression | |
| | **Initial** | **6M** | **Initial** | **6M** |
| | | (40° C/ 75% RH Aclar-Alu Blister) | | (40° C/ 75% RH Aclar-Alu Blister) |
| Total RS | 0.858 | 11.612 | 0.696 | 4.704 |

The above results show that changing the formulation process from direct compression to wet granulation for preparing compositions comprising unmicronised ropinirole increases the total related substances.

### Example 9: Ropinirole Composition and Stability Comparison

| **Example** | **Example 9** | **Example 4** |
|---|---|---|
| **Ingredients** | **mg/tab** | **mg/tab** |
| **Intragranular** | | |
| Ropinirole HCl | 0.285 | 0.285 |
| Lactose monohydrate (pharmatose 200M) | 38.715 | 15.0 |
| Microcrystalline cellulose | 35.0 | 70.0 |
| Lactose monohydrate (DCI 11) | - | 58.715 |
| Croscarmellose sodium | 4.5 | 4.5 |
| Purified Water | q.s. | - |

| **Extragranular** | | |
|---|---|---|
| Microcrystalline sodium | 35.0 | - |
| Lactose monohydrate (DCI 11) | 35.0 | - |
| Magnesium stearate | 1.5 | 1.5 |
| **TOTAL** | 154.5 | 150 |
| Opadry film coating | 4.5 | 4.5 |
| **Particle size of ropinirole HCl** | **Unmicronised*** | **Unmicronised**** |
| **Process** | **Wet Granulation** | **Direct Compression** |

| | | |
|---|---|---|
| *D₉₀=71µm, D₅₀ = 35µm, D₁₀= 9µ **D₉₀ = 67µm, D₅₀ = 30µm, D₁₀= 9µ | | |

### Process of Preparation for Composition of Example 9

1. Ropinirole, lactose (lactose 200M), microcrystalline cellulose, croscarmellose sodium and lactose (DCI 11) were all sifted and mixed in a rapid mixer granulator to obtain a blend
2. The blend obtained in step 1 was then granulated with purified water to obtain granules.
3. The granules of step 2 were dried and milled to desired size.
4. Microcrystalline sodium, lactose monohydrate and magnesium stearate were sifted through suitable sieve and mix with the granules obtained in step 3 to obtain a blend.
5. The blend obtained in step 4 was then compressed using appropriate toolings to obtain tablets.
6. The tablets obtained in step 5 were then coated with 12%w/v dispersion of Opadry to obtain coated tablets.

The compositions of Example 9 and Example 4 were evaluated for total related substances (both at initial time point and after storage). The results are provided in Table 6 below.

**Table 6: Comparison of Compositions of Example 9 and Example 4**

| | **Example 9** | | **Example 4** | |
|---|---|---|---|---|
| **Particle size of ropinirole HCl** | **Unmicronised** | | **Unmicronised** | |
| **Process** | Wet granulation | | Direct Compression | |
| | **Initial** | **15D** (60°C,HDPE bottle) | **Initial** | **15D** (60°C,HDPE bottle) |
| Total RS | 0.242 | 0.539 | 0.08 | 0.205 |

The above results further show that compositions prepared by direct compression have lower total related substances than compositions prepared by wet granulation.

Ropinirole hydrochloride, both micronised and unmicronised, was evaluated for total related substances (both at initial time point and after 6 months storage). The results are provided in Table 7 below:

**Table 7:**

| Ropinirole Hydrochloride | | | | |
|---|---|---|---|---|
| **Particle size of ropinirole HCl** | **Micronised*** | | **Unmicronised**** | |
| | **Initial** | **6M** (40° C/ 75% RH HDPE bottle) | **Initial** | **6M** (40° C/ 75% RH HDPE bottle) |
| Total RS | 0.185 | 0.176 | 0.185 | 0.165 |

| | | | | |
|---|---|---|---|---|
| * D₉₀ = 6 µm, D₅₀= 3 µm, D₁₀= 1µm ** D₉₀ = 108 µm, D₅₀= 40 µm, D₁₀= 16µm | | | | |

As evident from the above data, ropinirole hydrochloride is stable in both micronised and unmicronised state with respect to the total related substances.

It was further observed that the stable ropinirole composition according to the present invention had better stability when compared to marketed Requip^{®} tablets from GlaxoSmithKline. The amount of impurities (at initial time point and after 6 months) is provided in table 8 below:

**Table 8:**

| **Example** | **Requip^{®} Tablets 0.25mg** | | **Example 4** | |
|---|---|---|---|---|
| | **Initial** | **6M** (40° / 75% RH, Aclar-Alu Blister) | **Initial** | **6M** (40° / 75% RH, Aclar-Alu Blister) |
| Impurity 1 | 0.096 | 0.8 | ND | 0.229 |
| Impurity 2 | 0.83 | 0.4 | 0.039 | 0.301 |
| Impurity 3 | 0.09 | 0.25 | ND | 0.011 |
| Impurity 4 | - | 0.5 | ND | 0.218 |

The above results further show that compositions having unmicronised ropinirole and prepared by direct compression have lower amounts of impurity in comparison to Requip^{®} tablets.

## Claims

**1.** A stable ropinirole composition comprising unmicronised ropinirole and one or more pharmaceutically acceptable excipients.

**2.** The composition according to claim 1, wherein the unmicronised ropinirole has a particle size having a D₉₀ value from 50 to 200 microns, a D₅₀ value from 20 to 80 microns and a D₁₀ value from 5 to 30 microns.

**3.** The composition according to claim 1, wherein the one or more pharmaceutically acceptable excipients are selected from one or more of binders, diluents, disintegrants, lubricants or mixtures thereof.

**4.** The composition according to claim 3, wherein the binders are selected from one or more cellulose derivatives, gums, water-soluble vinylpyrrolidone polymers, sugars or mixtures thereof.

**5.** The composition according to claim 3, wherein the diluents are selected from one or more sugars, sugar alcohols, cellulose derivatives, starches or mixtures thereof.

**6.** The composition according to claim 3, wherein the disintegrants are selected from one or more sodium starch glycolate, croscarmellose sodium, crospovidone and corn starch.

**7.** The composition according to claim 3, wherein the lubricants are selected from one or more of magnesium stearate, talc, sodium stearyl fumarate and colloidal silicon dioxide.

**8.** The composition according to claim 1 prepared by direct compression.

**9.** A stable ropinirole composition comprising ropinirole and one or more pharmaceutically acceptable excipients wherein the composition is devoid of colloidal silicon dioxide.

**10.** The composition according to claim 9, wherein the composition is devoid of talc.

**11.** The composition according to claim 9, wherein the ropinirole is unmicronised ropinirole.

**12.** The composition according to claim 11, wherein the unmicronised ropinirole has a particle size having a D₉₀ value from 50 to 200 microns, a D₅₀ value from 20 to 80 microns and a D₁₀ value from 5 to 30 microns.

**13.** The composition according to claim 9 prepared by direct compression.

**14.** The composition according to claim 9, wherein the one or more pharmaceutically acceptable excipients are selected from one or more binders, diluents, disintegrants, lubricants or mixtures thereof.

**15.** The composition according to claim 14, wherein the binders are selected from one or more cellulose derivatives, gums, water-soluble vinylpyrrolidone polymers, sugars or mixtures thereof.

**16.** The composition according to claim 14, wherein the diluents are selected from one or more sugars, sugar alcohols, cellulose derivatives, starches or mixtures thereof.

**17.** The composition according to claim 14, wherein the disintegrants are selected from one or more of sodium starch glycolate, croscarmellose sodium, crospovidone and corn starch.

**17.** The composition according to claim 14, wherein the lubricants are selected from one or more of magnesium stearate, talc, sodium stearyl fumarate and colloidal silicon dioxide.

**19.** A process for the preparation of a stable ropinirole composition comprising the steps of:
(a) preparing a dry blend of ropinirole and one or more pharmaceutically acceptable excipients, and
b) directly compressing the dry blend of step (a) to form a stable ropinirole composition.

**20.** The process according to claim 19, wherein the ropinirole is unmicronised ropinirole.
